# EUROPEAN PATENT APPLICATION

(11) **EP 1 847 261 A1**
(43) Date of publication of application: **24.10.2007**
(21) Application number: 06708855.9
(22) Date of filing: 27.01.2006
(51) Int. Cl.: A61K 9/50

(54) **ASSEMBLY OF DEVICES USED FOR THE PRODUCTION OF MICROCAPSULES FOR THE CONTROLLED RELEASE OF PEPTIDES**

(30) Priority: 28.01.2005 AR P050100302
(71) Applicant: Laboratorio LKM S.A., Buenos Aires (AR); Sanjurjo Morales, Mario Daniel, Buenos Aires (AR)
(72) Inventor: ENRIQUEZ, Graciela, Buenos Aires (AR); HAYDEE MARRONE, Graciela, Buenos Aires (AR); GRASSI, Fernando, Daniel, Buenos Aires (AR); RAMAT, Cristina Beatriz, Buenos Aires (AR)
(74) Representative: Durán Moya, Luis-Alfonso
(86) International application number: PCT/ES2006/000036
(87) International publication number: WO 2006/082265

(57) **Abstract**

The invention relates to an arrangement of devices that can be used for the production of microcapsules for the controlled release of peptides, which optimises the entrapment of said peptides. The invention consists of a closed sheathed container comprising a built-in system for stirring and/or homogenising the contents thereof in a controlled manner. According to the invention, the container is communicated with a second container by means of an injection device with a diameter of less than 1 mm, said second container having similar characteristics to the first and being used to perform a second homogenization operation. The inventive arrangement of devices can be used to ensure a strict control of the temperature and the viscosity of the contents thereof, such that the temperature is always maintained below or equal to 15°C and the viscosity is maintained in a range of between 5000 and 20000 cp. In addition, the temperature and viscosity control system enables the size distribution of the microspheres obtained to be controlled.

## Description

### Technical field of the invention

The current invention refers to an assembly of devices used for the production of microcapsules for the controlled release of peptides, which optimises the entrapment of said peptides. Thus, the invention is related to useful devices and procedures in manufacturing microcapsules of biodegradable and biocompatible polymeric materials containing at least one active peptide.

### Background of invention

The development of controlled release formulations of drugs has taken an important impulse in recent years. Particularly, due to the possibility of effectively controlling the release of drugs, the controlled release formulations of parenteral use involving biodegradable polymers has taken great importance. Among these polymers -given their availability, biodegradability, non-toxicity and biocompatibility- the lactic and glycolic acids have played an important function.

Among these formulations the microspheres are included, which have been successfully used to encapsulate a wide variety of active ingredients, such as, cytostatics, anti-inflammatory agents, peptides and hormones. These microspheres are spheric analogous particles that do not present a clear distinction between nucleus and wall. They have a monolytical structure prepared from biodegradable materials and present a wide spectrum of delivery speed and degradative properties, where the active ingredient or therapeutical agent is distributed through the matrix as a molecular dispersion or a dispersion of particles.

Several methods for the preparation of microspheres of different biodegradable polymers have been used, including natural and synthetic polymers. The choice of a proper preparation method depends on the polymers properties and the active ingredient that is used and may affect the characteristics of the microspheres.

The systems based in the biodegradable polymer acid poli(D,L-lactic-co-glycolic) (PLGA), specially in the form of microspheres for injections, are currently under research for the development of new products of controlled release of proteins and pharmacologically active peptides.

On the other hand, the microcapsules have found application in a wide number of fields, among others, pharmaceutical, biomedical, agrochemical, food industry, cosmetic industry, photographic, cleaning products and graphic arts products.

The microencapsulation may be considered as a special way for packing, in which a particular material may be covered individually to be protected from the environment and harmful influences. In the broad sense, the micro-encapsulation provides a means for packaging, separating and storaging materials in a microscopic scale for their subsequent release under controlled conditions. Within the term of micro-encapsulation, the microcapsules, microparticles, nanocapsules and active substances trapped or imbedded are included, although there is a specific terminology depending on the application industry; for example, the pharmaceutical industry makes a distinction between microcapsules and microspheres, depending on how the material encapsulated within the particle is distributed.

The micro-encapsulation is a process in which tiny particles or droplets are surrounded by a coating, to obtain capsules of different sizes that can vary within the range from micrometers to millimeters. The capsules thus obtained are known as "microcapsules". The material inside the microcapsule is referred to as the core, internal part, nucleus or fill, whereas the coating is called external part, wall or membrane.

The microcapsules present a broad variety of structures, some of them are spherical geometry with an internal continuous phase surrounded by a wall, which is also continuous (simple particle structure), whereas others may have an irregular geometry and may have the internal part distributed in a matrix of wall material (added structures) (Shahidi, F., Han, X., 1993. "Encapsulation of Food Ingredients. Critical Reviews in Food Science and Nutrition" 33 (6): 501-547; Thies, C., 1996. "A survey of Microencapsulation proceses". En: Microencapsulation. (ed. By S. Benita), pp.1-20. Marcel Dekker, Inc. N.Y., EUA.), and although there are different points of view as regards the size interval to which they belong, it could be said that they range from 0.2 to 5000 µm (Luzzi, L., 1970. "Microencapsulation". J. Pharmaceutical Sci. 59(10): 1367-1376.; Bakan, J., 1973. "Microencapsulation of foods and related products ". Food Technology November. Pp. 34-44.; King, A., 1995. "Encapsulation of food ingredients". En: "Encapsulation and controlled release of food ingredients" (ed. by S. Risch and G. Reyneccius), pp. 26-39. ACS Symposium Series 590, American Chemical Society, Washington D.C.).

The advantages of microcapsules are in itself evident: fluids may be handled as solids, the smell or taste may be effectively mask in a food product, the filling substances may be protected from the harmful environment surrounding them, the toxic materials may be safely handled and the release of therapeutical drugs may be carried out in a sustained way.

The micro-encapsulation is an interdisciplinary field that requires knowledge regarding the polymers science, the emulsion technologies (in many cases) and the understanding of the stabilization of active ingredients. Perfumes and fragrances have been encapsulated from 1930, vitamins from 1940 and the paper ink without coal from 1956 *(*Encyclopedia of Controlled Drug Delivery, vol. 2, Ed. John Wiley, 1999, pp. 493-546). The concept of the use of semi-permeable microcapsules for the release of therapeutical reactive agents was introduced by T.M.S. Chang approximately 40 years ago (Chang, T.M.S., Science 146 (1964) pp. 524-525) and through the years, a broad spectrum of drugs, such as steroids, vitamins and antibiotics have been encapsulated.

For example, the micro-encapsulation has been extensively used within the detergents industry. Some detergent powders have reactive enzymes to the proteins (proteases), used to eliminate the blood stains. Due to aesthetic and safety reasons when handling, the enzymes are encapsulated in polymers soluble in water, such as polyethylene glycol. Once the external part is dissolved in the washing machine, the enzymes are released and react with the blood proteins, thus removing the stains.

Within the food industry, many commercial mixtures of preparations to be baked include encapsulated ingredients to generate chemical reactions at the moment of reaching the proper temperature. The sodium bicarbonate is an ingredient of these mixtures that reacts with the acids present in the food to produce leavening agents, which provides the proper volume and the spongy texture to the baked products. In order to control the leavening process, the sodium bicarbonate is encapsulated in grease, solid at room temperature, but it melts at approximately 52°C. In this way, the release of the material within the product is postponed until the mixture reaches the proper temperature.

The micro-encapsulation of products for the pharmaceutical industry is especially useful when a sustained release is required over time. The release speed of a bioactive substance to an organism or reaction site is critical many times. The key advantages of the use of this technology are the prolonged activity, the fewer amounts of dose, fewer amounts of adverse effects and the decrease of toxicity. A large amount of a consumed or injected pharmaceutical product at the same time in order to achieve a given concentration means a material that has been lost uselessly or toxic adverse effects. Reducing the dose's speed in order to avoid these problems, better efficacy results may be achieved. One of the main purposes in the controlled release is to determine the best release's speed in order to achieve an ideal performance.

For example, the aspirin is effective to alleviate the fever, inflammation and arthritis, but direct doses of aspirin may cause peptic ulcers and bleeding. Therefore, many times this drug is encapsulated in ethyl cellulose or hydroxypropyl Methyl Cellulose and starch. In this way, instead of being released all together, the aspirin releases through its external covering slowly and in a sustained way.

The release systems consisting of microparticles may be administered by different paths, such as by means of standard needles and syringes, administered to the gastrointestinal system and the nasal tissue. However, the manufacturing processes of microparticles are often complex and difficult to control. Particularly, referring to costs and uniformity batch by batch. In order to form the external wall of the microcapsules, many polymers may be used with a main hydrophobic chain that degrade in biocompatible waste materials. The biodegradable polymers more broadly used are the lactic and glycolic acid, the ones of the caprolactones and hydroxybutyrate, as well as combinations of these and other polymers. Both the poli(d,l,-lactic) (PLA) acid, as well as the poli(d,l,-lacticglycolic) (PLGA) acid, which is a copolymer from the d,l-lactic acid and the glycolic acid, are the polymers more broadly used for medicinal purposes.

There are several methods for the preparation of biodegradable microspheres prepared using polymers from lactic and glycolic acid. The methods of evaporation/extraction of the solvent and phases separation are the 2 main methods used for these purposes.

The choice of the micro-encapsulation technique carried out, mainly, on the grounds of the polymer's physico-chemical characteristics and active ingredient to be encapsulated. Thus, the following requeriments shall be taken into account:
- The performance of microspheres within the desired range of sizes should be high.
- Encapsulation's efficiency of the active ingredient should be high.
- The biological activity of the active ingredient should remain during the process of encapsulation.
- The batch by batch reproducibility in terms of a qualitative profile and the release of the active ingredient should be within the specified limits.
- The release profile should be adjusted by means of the control of the composition and process' variables.
- The microspheres should not be added, they should be a fine powder that flows freely.

On the other hand, some of the microspheres properties that should be optimized are the following:
- Size and distribution of sizes.
- Surface properties.
- Load of active ingredient.
- Release speed of the active ingredient.
- Degradation speed of the matrix.

Other aspects as sterility, apyrogenicity and residual solvent content have to be satisfactory.

The preparation of a microencapsulated product involves several steps. First of all, the need for micro-encapsulation should be identified, for example if we need to improve the quality of an existing product or if we wish to develop an entirely new product. Afterwards, a material for the external coating should be identified that provides the needs for the desired release. Finally, a procedure to prepare the microcapsules should be chosen.

The choice of the micro-encapsulation technique is carried out, mainly, on the grounds of the physico-chemical characteristics of the polymer and the active ingredient to be encapsulated. Some of the important requirements to be taken into account are that the performance of microcapsules with the desired sizes interval and the encapsulation's efficiency of the active ingredient should be high. The biological activity of the active ingredient should remain during the process of encapsulation and both the batch by batch reproducibility in terms of a qualitative profile and the release of the active ingredient should be within the specified limits. Moreover, the release profile should be adjusted by means of the control of the composition and process' variables and the microcapsules should not be added; they should be a fine powder that flows freely.

In order to prepare the microcapsules, there are many techniques and it has been suggested that more than 200 methods in the literature of patents may be identified (Magdassi, S., Vinetsky, Y., 1996. "Microencapsulation of oil-in-water emulsions by proteins". En: Microencapsulation. (ed. by S. Benita), pp. 21-34. Marcel Dekker, Inc. N.Y., EUA. and Brazel, C. S., 1999. "Microencapsulation: Offering solutions for the food industry". Cereal Foods World 44(6): 388-393., 1999). However, some authors classify the encapsulation methods in: physical or mechanical and chemical.

The two main methods used for the preparation of microcapsules are the separation of phase's method and the solvent evaporation/extraction techniques. Other methods that can be mentioned are: drying by atomization, hot fusion, cold drying and fluidization.

The separation of phases method (named as well coacervation) is a non- aqueous method for preparing microcapsules and is used, mainly, for active ingredients soluble in water. An aqueous solution of the drug, or the drug as powder, is dispersed in an organic solvent in which the polymer was dissolved previously. Once the emulsion is formed, a coacervant agent is added (generally a vegetal or mineral oil that functions as nonsolvent for the polymer) inducing the formation of the microcapsules containing the active ingredient. The main disadvantages of this method are the frequent agglomeration, due to the absence of a stabilizer, the batch by batch variation, which may cause problems both in the coacervation process, as well as in the release speed's profile and its greatest cost, compared with the other methods due to the huge amount of solvents needed, increasing, in turn, the environmental pollution.

In the method of solvent evaporation/extraction technique, all of the processes where this last process is eliminated are included -in which the polymer is dissolved - whether it is by evaporation or by its extraction. In all cases, an emulsion has to be formed previously. According to the nature of the continuous phase of the emulsion that is formed, they shall be classified in techniques of solvent evaporation/extraction in aqueous phase or in oily phase.

In aqueous phase there are two main methods, the one of emulsion oil in water (o/w) and the emulsion water in oil in water (w/o/w), known as well as multiple emulsion.

In the first one of these two methods (o/w), the organic phase containing the polymer and the active ingredient are emulsified in an aqueous phase containing a tensioactive agent. Subsequently, the organic emulsified particles containing the polymer and the active ingredient are hardened as microcapsules by elimination of the organic solvent. Some of the advantages of this method are the efficient addition of lipophilic active ingredients, the wide range of sizes of microcapsules that has been reached (basically controlled by the speed and stirring conditions) and the hydrophilic superficial properties of the microcapsules, which allows its resuspension without addition. The main method's disadvantage is that the addition of active ingredients soluble in water is very low due to the distribution of the active ingredient in the external aqueous phase of the emulsion.

The second method in aqueous phase (w/o/w) is a modification of the o/w method, which is used to encapsulate active ingredients soluble in water and has proved to be very efficient to encapsulate this type of substances. In this method, the active ingredient is dissolved in water (aqueous phase) and the polymer is dissolved in an organic solvent (organic phase). Both phases are mixed obtaining the w/o emulsion, which is slowly added on the aqueous medium containing an emulsifier such as the polyvinyl alcohol. Afterwards, the organic solvent is eliminated and the microcapsules achieved.

In the oily phase there is a main method, the % emulsion. This method is another modification of the o/w emulsion; the continuous phase is formed by an organic fluid such as the mineral oil and the emulsion is afterwards formed. It is used to efficiently encapsulate active ingredients soluble in water. The disadvantages of the % method are the difficulty to obtain tiny microcapsules (less than 50 µm) and that these tend to accumulate when they are resuspended in aqueous medium due to the hydrophobic nature of its surface and the absence of a hydrophilic stabilizer.

Other multiphase methods that have been reported more recently are the ones of w/o/w/o emulsion (Iwata M, McGinity JW, Pharm Res 1993;(10):1219-7), w/o/o emulsion (Yen MK, Coombes AGA, Jenkins PG, Daris SS, J Controlled Rel 1995(33):437-5) and w/o/o/o emulsión (Iwata M, McGinity JW., J Microencapsulation 1992(9):201-14).

Although the solvent evaporation/extraction technique above described is simple, there exist many variables that may have influence in the characteristics of the microcapsules that have been obtained. These variables are the organic solvent, the nature and amount of the emulsifier, temperature of solvent's evaporation, volume relation of the organic and aqueous phases, active ingredients and polymer relation, polymer's structure and molecular mass, type and speed of stirring.

### Brief description of the invention

The invention refers to an assembly of devices used for the production of microcapsules for the controlled release of peptides, which optimises the entrapment of said peptides, characterized because it consists of a closed sheathed container comprising a built-in system for stirring and/or homogenising the contents thereof in a controlled manner, where such container is communicated with a second container by means of an injection device with a diameter of less than 1 mm, said second container having similar characteristics to the first and being used to perform a second homogenisation operation and because such assembly of devices can be used to ensure a strict control of the temperature and the viscosity of the contents thereof, such that the temperature is always maintained below or equal to 15 °C and the viscosity is maintained in a range of between 5000 and 20000 cp and, in addition, the temperature and viscosity control system enables the size distribution of the microspheres obtained to be controlled.

Moreover, the invention refers as well to a procedure to manufacture microcapsules for the controlled release of peptides, which involves:
- preparing a first aqueous phase (W₁) solubilizing a polymer in water and solubilizing the hydrophilic peptide in such aqueous phase,
- preparing an organic phase (O) of a proper solvent,
- emulsifying the aqueous phase (W₁), at a temperature approximately 2°C-10°C, in the oily phase (O), to acquire an emulsion W₁/O).
- dispersing the emulsion been obtained in the previous stage in the aqueous phase of the covering colloid (W₂), at a temperature within the range of 15°C-20°C, until obtaining an emulsion W₁/O/W₂,
to finally evaporate the solvent at a temperature within the range of 25°C-40°C and subsequent centrifugation and lyophilization in order to obtain the microparticles afterwards.

### Figures' brief description

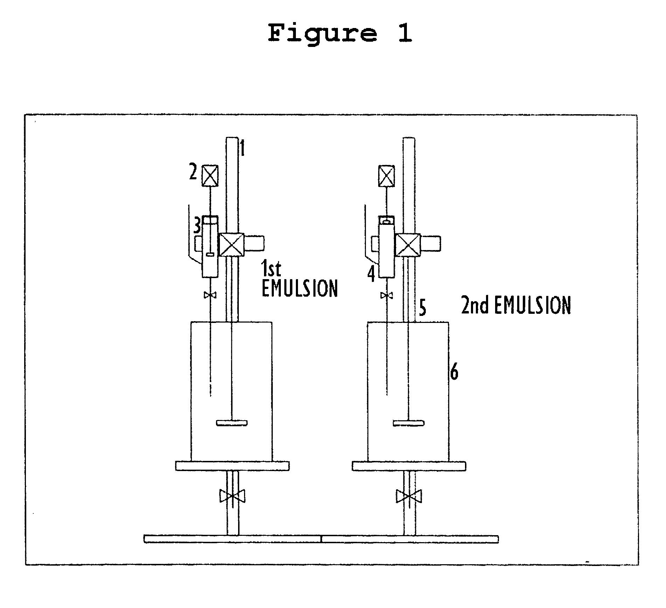

In figure 1 there appears a scheme of the assembly used in manufacturing of microcapsules for the controlled release of peptides corresponding to the invention.

### Description of the invention

We have found that the temperature of evaporation has an influence on the peptide release's profile. Thus, by controlling the temperature, we were able to obtain profiles of similar release, using polymers of different molecular weights and different lactic- glycolic relation.

In particular, this is one of the purposes of the current invention, an assembly of devices used for the production of microcapsules for the controlled release of peptides, which optimises the entrapment of said peptides, which by means of the temperature and viscosity control enables the size distribution of the microspheres obtained to be controlled.

The previous art processes have low performance. Therefore, we have designed a device that minimizes the losses due to the transfer of containers, which allows obtaining improved performances of approximately 70%.

Thus, one of the purposes of the invention consists in an assembly of devices used for the production of microcapsules for the controlled release of peptides, which optimises the entrapment of said peptides, characterized in that it consists of a closed sheathed container comprising a built-in system for stirring and/or homogenising the contents thereof in a controlled manner, where such container is communicated with a second container by means of an injection device with a diameter of less than 1 mm, said second container having similar characteristics to the first and being used to perform a second homogenisation operation and in that such assembly of devices can be used to ensure a strict control of the temperature and the viscosity of the contents thereof, such that the temperature is always maintained below or equal to 15 °C and the viscosity is maintained in a range of between 5000 and 20000 cp and, in addition, the temperature and viscosity control system enables the size distribution of the microspheres obtained to be controlled.

Preferably, the device of the invention may include a plunger, activated electric or hydraulically, which pushes the emulsion and minimizes the loss of product that would happen if the product adheres to the wall of the container.

Particularly, the invention refers to an assembly of devices used for the production of microcapsules for the controlled release of peptides soluble in water, which optimises the entrapment of said peptides, characterized because it consists of a closed sheathed container comprising a built-in system for stirring and/or homogenising the contents thereof in a controlled manner, where such container is communicated with a second container by means of an injection device with a diameter of less than 1 mm, said second container having similar characteristics to the first and being used to perform a second homogenisation operation and because such assembly of devices can be used to ensure a strict control of the temperature and the viscosity of the contents thereof, such that the temperature is always maintained below or equal to 15 °C and the viscosity is maintained in a range of between 5000 and 20000 cp and, in addition, the temperature and viscosity control system enables the size distribution of the microspheres obtained to be controlled. Preferably, the temperature remains within a range of 3-10°C and the viscosity remains within a range of 10,000 and 20,000 cp.

As regards the solvents used in the solvent evaporation/extraction technique, the solvent for the polymers of lactic/glycolic acid should be immiscible or slightly soluble in the suspension medium (aqueous or oily). Additionally, the solvent's boiling point should be lower than the one of the suspension medium if the solvent is eliminated by evaporation. Solvents more commonly used are ethyl acetate and dichloromethane, due to its low toxicity, its easy elimination and its excellent capacity to dissolve polymers. Other solvent used have been the chloroform and acetonitryl. If what is desired is the dissolution of the active ingredient in the polymer's solution, it is also needed to take into account the capacity of the solvent to dissolve. Mixtures of solvents have also been used to dissolve the polymer and the active ingredient, for example, dichloromethane (immiscible in water) plus methanol, ethanol or Propylene glycol (miscible in water). The use of these last three allows a fast elimination of the solvent and a fast polymer's precipitation.

The emulsifiers provide a fine covering sheet surrounding the oil droplets, polymer and active ingredient and, in this way, the coalescence and coagulation decrease and stabilize the emulsion system. Frequently, the initial difficulty found in the development of a micro-encapsulation procedure is the agglomeration of the oil droplets during the process of manufacture. The emulsifiers more commonly used in the process of solvent's evaporation/extraction are the hydrophilic polymeric colloids and the anionic or non ionic surfactants. Some examples are: polyvinilic alcohol, la Polyvinylpyrrolidone, los alginates, gelatin, methylcelullose, hydroxyalkylcellulose, polysorbate, span, lecithin, etc. The more commonly used emulsifier used in the o/w method is the polyvinilic alcohol. The required concentration and effectiveness of each emulsifier is different and the best emulsifier for one application in particular may be chosen by experiments.

The polymer's nature, the sequence of the monomers and, thus, its molecular mass allow adjusting the drug's release, while the therapeutic blood concentrations are reached. The inherent viscosities of polymers of different molecular masses tend to vary, this parameter determining the efficiency of the process of production of the microspheres and their properties.

The rest of the parameters have an influence in the optimization process of the production method and are intimately related to the solvent, polymer, emulsifier and the active ingredient.

It is particularly interesting to this current invention the encapsulation procedure that the "in-water drying" process uses, which is a particular case of the already mentioned w/o/w emulsion. The advantage of this method is that it is very versatile, since it does not need too specific conditions, any need to adjust the pH, or any special reactive. In this way, it is possible to use both stable and unstable materials.

Preferably, the invention procedure is an intermittent procedure. This is a clear difference regarding the procedure revealed in the US patent 6,534, 094. In fact, all of the teachings of this last patent would lead the expert in the field to not try to develop a procedure discontinuously and, much less, with the characteristics of invention. However, the continuous process revealed in the US 6,534,094 requires the use of very low viscosities, which finally results in important losses of the finished product. On the contrary, due to the use of higher viscosities, the invention procedure allows to acquire better manufacturing performances.

The microcapsules containing pharmaceutically active substance that may be soluble in water or that may form a suspension in aqueous phase is particularly interesting to this current invention. The peptides soluble in water are preferably interesting as active ingredients and specially the hormones. More interesting is the leuprorelin acetate. Leuprorelin belongs to the class of drugs known as gonadotrophin hormone-releasing hormone (LH-RH). It is similar to a hormone normally released by the hypothalamus and stops the production of testosterone in men and estrogen in women. This blocking may be desired since the testosterone reduction in the body is a way of treating the prostate cancer, while the reduction of estrogens is a way of treating the endometriosis. By means of the reduction of tumors in the uterus, leuprorelin helps stopping the anemia, since it reduces the vaginal bleedings that cause these tumors. Leuprorelin is also indicated in children (boys and girls) enduring early puberty, since it reduces the development of the genital areas in both sexes and the development of breast in girls.

Preferably, the device and the procedure of the invention result to be specially used with peptides soluble in water, although they could also be used with lipophilic peptides. In this way, the leuprolide acetate is the most preferably of the group involving the peptides soluble in water, and the paclitaxel is the most preferably of the group involving the peptides insoluble in water.

Other hormones/drugs/ active ingredients that are specially interesting regarding this current invention are, for example: leuproreline, goserelin, triptorelin, nafarelin, buserelin, oxytocin, insulin, interferon, interleukin, etc.

### Examples of execution

### Production process of controlled release microparticles:

The process begins with the preparation of three phases:
- An aqueous phase (W₁) formed by the hydrophilic peptide, which is solubilized in water and gelatin,
- an organic phase (O) formed by the biodegradable PLGA polymer solution in dichloromethane (DCM)
- and another aqueous phase (W₂) of a covering colloid, in this case, polyvinyl alcohol.
- Subsequently, the aqueous phase (W₁) is emulsified by means of a homogenizer in the oily phase (O) that results in (W₁/O). The emulsion acquired is dispersed by means of a homogenizer in the aqueous phase of the covering colloid (W₂) obtaining the W₁/O/W₂ emulsion.

Once the second emulsion (W₁/O/W₂) is stabilized, the next step is the evaporation of the DCM (dichloromethane) in order to obtain the microparticles. This procedure is carried out by stirring the emulsion and keeping it a constant temperature for an hour.

The microparticles that have been obtained are separated from the supernatant liquid by centrifugation and are washed with water. Finally, they are dried by lyophilization.

The procedures involved are following described:

### DISPERSION (W₁)

483 mg of gelatin are weighed in a container. In the container R1 the gelatin is dispersed in 3ml of water at room temperature. It is later placed in bath at 40°C. 2.7 g of leuprolide acetate are added, and then it is allowed to cool and remain with a temperature lower than 10°C.

### DISOLUTION 2 (O)

24 g of the PLGA polymer are weighed and dissolved with mechanical agitator in such an amount of dichloromethane that it is able to acquire the proper viscosity. The necessary amount of solvent depends on the polymer's inherent viscosity. Viscosity of the polymer's solution is controlled with temperature.

### DISOLUTION 3 (W₂)

A solution of polyvinyl alcohol (Mowiol 40/80) is prepared at 0.5 %, it is allowed to cool and remain at 15°C until it is used.

### HOMOGENIZATION 1 (W₁/O)

O is added to W1. This procedure is carried out at a temperature lower than 15°C. It is then left to rest during one hour, approximately, until the viscosity is approximately 10000 cps, which is a measure with a rotational viscometer.

### HOMOGENIZATION 2 (W₁/O)/W₂

Container R1 receives the plunger that has been added and the needle. W1/O is slowly added in W2 at 15°C, stirring at 20,000 rpm. Once W₁/O has been added, the stirring continues for 5 more minutes.

### EVAPORATION

The emulsion been obtained in the previous step is stirred with the mechanical agitator, with propeller at 2000 rpm, for 1 hour at room temperature.

### CENTRIFUGATION AND WASHING

It is centrifuged at 5000 rpm, during 15 minutes at 15°C, rejecting the supernatant liquid and is washed three times with 450 ml of Distilled water. After the second wash, it is filtered with a mesh of 80µ-pore size. Approximately 450 ml of water are used to drag the retained microspheres. It is centrifuged once again under the same conditions. Mannitol at 2 % is added and afterwards, lyophilized.

### List of numerical references

- 1: Support of the device
- 2: Emulsifier
- 3: Container for first emulsification
- 4: Injection system with moving plunger
- 5: Emulsifier with container

## Claims

1. An assembly of devices used for the production of microcapsules for the controlled release of peptides, which optimizes the entrapment of said peptides, **characterized in that** it consists of a closed sheathed container comprising a built-in system for stirring and/or homogenizing the contents thereof in a controlled manner, where such container is communicated with a second container by means of an injection device with a diameter of less than 1 mm, said second container having similar characteristics to the first and being used to perform a second homogenization operation and **in that** such assembly of devices can be used to ensure a strict control of the temperature and the viscosity of the contents thereof, such that the temperature is always maintained below or equal to 15 °C and the viscosity is maintained in a range of between 5000 and 20000 cp and, in addition, the temperature and viscosity control system enables the size distribution of the microspheres obtained to be controlled.

2. An assembly of devices used for the production of microcapsules for the controlled release of peptides soluble in water, which optimizes the entrapment of said peptides, **characterized in that** it consists of a closed sheathed container comprising a built-in system for stirring and homogenizing the contents thereof in a controlled manner, where such container is communicated with a second container by means of an injection device with a diameter of less than 1 mm, said second container having similar characteristics to the first and being used to perform a second homogenization operation and because such assembly of devices can be used to ensure a strict control of the temperature and the viscosity of the contents thereof, such that the temperature is always maintained below or equal to 15 °C and the viscosity is maintained in a range of between 5000 and 20000 cp and, in addition, the temperature and viscosity control system enables the size distribution of the microspheres obtained to be controlled.

3. An assembly of devices used for the production of microcapsules for the controlled release of peptides, according to any of the previous claims, **characterized in that** it consists, moreover, of a plunger activated electric or hydraulically, which pushes the emulsion and minimizes the loss of product that could happen if the product remained attached to the container's walls.

4. An assembly of devices used for the production of microcapsules for the controlled release of peptides, according to any of the previous claims, **characterized in that** the temperature is maintained in a range of between 3- 10°C.

5. An assembly of devices used for the production of microcapsules for the controlled release of peptides, according to any of the previous claims, **characterized in that** the viscosity is maintained in a range of between 10000 and 20000 cp.

6. A procedure used for the production of microcapsules for the controlled release of peptides **characterized in that** it comprises:
the preparation of a first aqueous phase (W₁) solubilizing a polymer in water and the solubilization of the hydrophilic peptide in such aqueous phase,
the preparation of an organic phase (O) of a proper solvent and by means of the use of the device, according to claim N° 1,
the emulsion of the aqueous phase (W₁), at a temperature approximately of between 2°C-10°C, in the oily phase (O), in order to obtain an emulsion (W₁/O),
dispersing the emulsion obtained in the previous stage in the aqueous phase of the covering colloid (W₂), at a temperature within the range of 15°C and 20°C, until achieving an W₁/O/W₂ emulsion,
to, finally, evaporate the solvent at a temperature within the range of 25°C and 40°C and subsequent centrifugation and lyiophilization so that afterwards the microparticles can be obtained.

7. A procedure used for the production of microcapsules for the controlled release of peptides, according to the previous claim, **characterized in that** it is an intermittent procedure.

8. A procedure used for the production of microcapsules for the controlled release of peptides, according to the previous claim, **characterized in that** the peptides are selected among those who are soluble in water.

9. A procedure used for the production of microcapsules for the controlled release of peptides, according to the previous claim, **characterized in that** the peptide is leuprolide acetate.

10. A procedure used for the production of microcapsules for the controlled release of peptides, according to the previous claim, **characterized in that** the peptides are selected among those who are insoluble in water.

11. A procedure used for the production of microcapsules for the controlled release of peptides, according to the previous claim, **characterized in that** the peptide is paclitaxel.
